# EUROPEAN PATENT SPECIFICATION

(11) **EP 1 412 815 B1**
(45) Date of publication and mention of the grant of the patent: **01.06.2005**
(21) Application number: 02751339.9
(22) Date of filing: 26.07.2002
(51) Int. Cl.: G03B 17/24

(54) **APPARATUS AND A METHOD IN THE FIELD OF PHOTOGRAPHY FOR RECORDING AND REPRODUCING SCENT OR TASTE**
VORRICHTUNG UND VERFAHREN AUF DEM GEBIET DER PHOTOGRAPHIE ZUR AUFNAHME UND WIEDERGABE EINES DUFTS ODER GESCHMACKS
Appareil et procédé d'enrégistrement et de réproduction de parfum ou d'arome pour la photographie

(30) Priority: 31.07.2001 GB 0118667
(43) Date of publication of application: 28.04.2004
(73) Proprietor: HEWLETT-PACKARD COMPANY, Palo Alto, California 94304-1181 (US)
(72) Inventor: MURPHY, Rachel, Lucy, Bristol BS4 2EU (GB); FROHLICH, David, Mark, Bristol BS9 3SS (GB)
(74) Representative: Jones, Bruce Graeme Roland
(86) International application number: PCT/GB2002/003440
(87) International publication number: WO 2003/011348

(56) References cited:
- EP-A- 0 844 781
- WO-A-99/16476
- DE-A- 19 928 592
- GB-A- 2 315 044
- US-A- 5 975 675
- US-A- 5 995 770
- US-A- 6 067 103
- US-B1- 6 229 565
- PATENT ABSTRACTS OF JAPAN vol. 2000, no. 09, 13 October 2000 (2000-10-13) -& JP 2000 156647 A (NIPPON TELEGR &TELEPH CORP <NTT>), 6 June 2000 (2000-06-06)
- PATENT ABSTRACTS OF JAPAN vol. 1998, no. 11, 30 September 1998 (1998-09-30) & JP 10 171067 A (ORIENTAL PHOTO IND CO LTD), 26 June 1998 (1998-06-26)

## Description

This invention relates to apparatus and a method for recording and reproducing scent or taste.

The fields of photography and film making have striven to develop increasingly more advanced and sophisticated techniques to capture a scene or event accurately and reproduce it realistically before an audience (e.g. in a cinema) or a viewer (e.g. of a still photograph). However, to date, only two of the five senses - hearing and vision - have properly been provided for. In order to reproduce accurately an experience before an audience or viewer it is necessary to go beyond sound and vision and provide stimuli directed to the other senses as well.

As yet, science and technology do not suggest any methods for readily conveying a sensation of touch to an audience, and this present work does not attempt to address this issue.

The senses of smell and taste contribute a great deal to the way we experience life. Furthermore, smells and tastes can serve as highly evocative reminders of past experiences. For example, the smells of exotic spices might remind one of a visit to a Turkish market. It would therefore be highly desirable to be able to convey smells and tastes along with the visual (and optionally audio) stimulus of a photograph or video film. Moreover, capturing smells and tastes at the point of taking the photograph would be desirable, since these would then be able to enhance the ability of the photograph to serve as a memento of the occasion.

Developments to date in the field of scent reproduction include the following: US 5,995,770 (Rochford et al.) discloses a system and method of delivering a scent to a photographic print during a photofinishing operation, and also a camera having a menu of scents from which a user may make a selection. This scent must be selected by the user from a limited list of alternatives, and is not necessarily representative of the scent of the location at which the photograph was taken. A method for capturing the local scent is not taught. A further disadvantage of this method is that it is not appropriate to those photographers who wish to obtain an instant picture (e.g. using a Polaroid (RTM) camera) or who wish to print their photographs themselves - e.g. from a digital camera, using a domestic inkjet printer (optionally in conjunction with a personal computer (PC)).

WO 00/15268 and WO 00/15269 (both in the name of Aromix Technologies Limited) state that gas chromatography or mass spectrometry techniques may be used to sense, identify or characterise odours through a process of chemical analysis. WO 00/15268 teaches that, having analysed an odour using such a technique, data specifying the odour may be transmitted to an output device (typically connected to a PC). WO 00/15269 discloses a method and apparatus for reproducing odours using concentration vector generators. These publications, however, do not teach the application of these techniques to photography.

US 6,152,829 (Jaidka) discloses apparatus for generating odour effects for use in a cinema or theatre, but this does not teach the application of scent to printed still photographs.

Other methods of sensing, analysing and recognising scents or odours, for example using solid state 'electronic noses', are well known in the art. Solid state electronic noses have been developed that use chemical sensors (e.g. based on metal oxide films) which measure a change in voltage due to the presence of certain chemicals in the air, and artificial neural networks may be used to enable the electronic nose to detect more chemicals than the number of sensors employed.

Techniques for the generation of odours through the mixing (and heating) of gases or liquids are also known in the fields of perfume and scented paper manufacture. Peripheral devices for attaching to PCs have been introduced by manufacturers such as DigiScents, Inc. (http://www.digiscents.com), and suggested (albeit in a spoof form) on the website http://www.realaroma.com. However, no attention has yet been given to the use of this technology in photographic applications.

As well as in amateur or domestic applications, there are also applications for aromatic photography (or 'aromaphotography') in commerce and industry.

The above-cited references do not disclose how tastes might be added to photographs. As with smell, taste is also highly evocative, and is particularly of relevance in conjunction with foodstuffs. For example, a conventional photograph taken of a meal is unable to provide the viewer with any appreciation of its taste or flavour. Potential applications for this technology would include cookbooks and advertising materials for food and drinks, as well as a means for generating a memento of a personal experience associated with taste.

It is a general object of the present invention to overcome or at least mitigate at least some of the shortcomings and problems identified above.

According to a first aspect of the invention there is provided a camera comprising image capture means operable to capture an image, integral scent association means operable by a user to associate a scent with the captured image, characterised by integral means operable to cause the said scent to be perceived by a user. The term "scent" as used herein should be interpreted broadly, encompassing both a smell as perceived by the nose and a taste as perceived orally. Such a camera may capture still or moving images, and optionally audio too.

Preferably the scent association means comprise a detector connected to the camera, the detector being operable to analyse the scent of its surrounding medium. The detector (which may alternatively be called a probe) may be integral with the camera, or electrically connected to it. The surrounding medium may be ambient air or a sample of liquid or solid. This feature advantageously enables the user to record the scent of his environment (or of the specific subject of the photograph) at the time of taking the photograph, or the taste of an object being photographed.

Particularly preferably the detector comprises a device selected from a group comprising a gas chromatography device, a mass spectrometry device, a chemical sensor. These devices provide known means by which scents may be detected or analysed.

Alternatively, preferably the scent association means comprise a user interface electrically coupled to processing means and a display, configured and operable to allow a user to select a scent from a menu. This has the advantage that the camera need not be provided with sophisticated scent detection apparatus. Instead the user can simply provide the camera with information as to the smell or taste of the object in question. A further advantage is that the user may specify a smell or taste that is not actually that of the subject of the photograph, but instead is associated with the object or scene for a more esoteric reason.

Preferably the camera is a digital camera and comprises transfer means operable to transfer an image and data representing the scent to processing means remote from the camera. Particularly preferably the transfer means are selected from a group comprising: a port configured to receive and electrically couple with a cable (e.g. a parallel, serial or USB cable), wireless digital communication means, infra-red digital communication means, radio frequency communication means. The processing means remote from the camera may be those of a printer or personal computer equipped with scent generation means. These features give the advantage that a photograph may be printed on a printer directly from a camera, the printer being equipped with means to generate the recorded scent. Alternatively the image may be viewed on the display of a PC, which triggers an attached scent-generating peripheral (e.g. as manufactured by DigiScents, Inc.) to generate the recorded scent.

This invention is of benefit to a wide range of people, including amateur photographers and hobbyists, detectives at a crime scene, research scientists, and workers in the food and drink industry such as wine tasters and brewers, all of whom may wish to capture a scent or taste together with a photographic image.

Preferably the camera further comprises a plurality of specimens of scents. This advantageously enables the user to release a scent or sample a taste at the point of taking a photograph. This scent need not be traditionally associated with the object of the photograph, but may be chosen so that subsequent detection of the scent or taste serves as an evocative reminder of the occasion of taking the photograph.

These specimens may correspond to the contents of the said menu and the camera may be configured to present a specimen to the user in accordance with a selection made using the said menu. This provides the advantages that the user is able to select a scent or taste using the menu and experience it at the point of taking a photograph.

Preferably the specimens are provided on a spool of tape operable by a user. Such a cassette may be loaded in to a camera and replaced when the specimens have been consumed. The cassette may also be transferred to another compatible device such as a printer, which may be configured to affix a scented specimen to a printed image during printing.

Particularly preferably each of the said specimens comprises a scented region covered by a scratchable coating, whereby scratching of the coating releases the scent from the underlying region. This enables the scent to be released as required by the user.

Alternatively each of the said specimens is edible.

Preferably the specimens are configured to be affixed to a printed photograph. Advantageously, therefore, the scent may be experienced by a user when viewing the photograph, the scent serving to provide the user with an enhanced recollection or appreciation of the object or event captured in the photograph.

To facilitate application of the specimens to photographs or other substrates, preferably the specimens have a self-adhesive surface.

Preferably the camera has an integral printer, thereby enabling substantially instant printing in a manner broadly analogous to that of a Polaroid (RTM) camera. Such cameras are useful for obtaining a picture straight away, without needing to print the photograph elsewhere at a later stage. Preferably such a camera is configured to affix a specimen onto a photograph on printing.

According to another aspect of the invention there is provided a method for delivering a scent with an image, comprising using a camera to capture an image and the further steps of: using the camera to associate a scent with the image; characterised by the step of using means integral with the camera to cause the said scent to be perceived by a user.

Preferably step (a) comprises using a detector connected to the camera to analyse the scent of the surrounding medium.

Altematively, step (a) may comprise operating a user interface to select a scent from a menu provided on a display on the camera.

Preferably a digital camera is used and the method further comprises transferring the image, and data representing the said scent, to processing means remote from the camera. Particularly preferably the method further comprises electronically transferring the image, and the data representing the said scent, to a device selected from a group comprising: a printer equipped with scent generation means, a personal computer equipped.with scent generation means; and then using the said scent generation means to generate the said scent for perception by a user.

Altematively the method further comprises providing a plurality of specimens of scents accessible from the camera from which the user can specify a choice.

Preferably the specimens correspond to the contents of a menu provided on a display on the camera, step (a) comprises operating a user interface to select a scent from the menu, and step (b) comprises the camera presenting a specimen to the user in accordance with the selection made by the user using the menu in step (a). In step (b) the specimens may be provided on a spool of tape accessible by the user, from which specimens the user may specify a choice. The method may further comprise transferring the spool of tape to a separate device from which the scent of the specified specimen may be perceived by a user, warming the specified specimen to cause the emission of its scent, or applying the selected specimen to the printed image, and ultimately a user smelling, licking or eating the specimen.

Embodiments of the invention will now be described, by way of example, and with reference to the drawings in which:
Figure 1 illustrates a rear view of a camera equipped with means for capturing and analysing scents, and a PC equipped with scent generation apparatus for reproducing the scent captured by the camera;
Figure 2 illustrates a rear view of a camera equipped with means for capturing and analysing scents, and a printer having printed an image with an attached scent;
Figure 3 illustrates a rear view of a camera equipped with means for capturing and analysing tastes;
Figure 4 illustrates a rear view of a camera equipped with a digital menu by which a used may specify a scent;
Figure 5 illustrates a rear view of a camera equipped with a cassette containing a plurality of specimens of scent;
Figure 6 illustrates a printer configured to receive the cassette from Figure 5 and to cause it to emit scents;
Figure 7 illustrates a front view of an instant camera having an integral printer and a spool of scented labels which may be applied to photographs;
Figure 8 illustrates a photograph having a scented region; and
Figure 9 illustrates a photograph having a plurality of edible tabs.

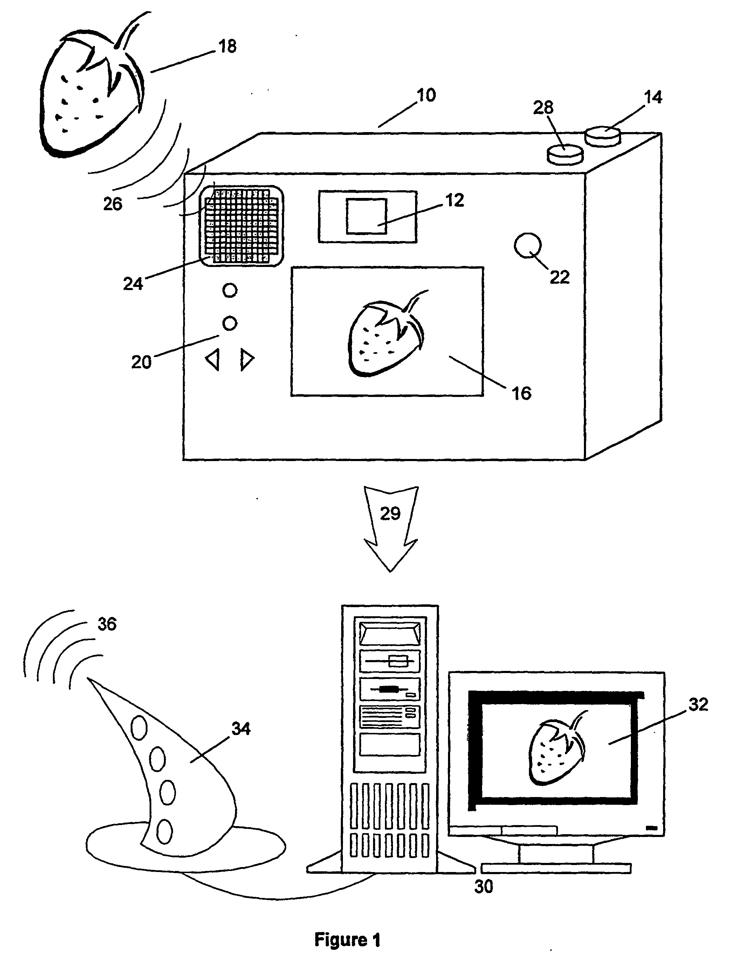

Figure 1 illustrates a digital camera 10, although the application of the present invention is by no means limited to digital cameras. The camera 10 comprises a viewfinder 12 for use in composing the photograph, an image capture button 14 for taking the picture, and a preview display panel 16 on the rear. In the example shown in the figure, the subject of the picture is a strawberry 18, and a preview of the image of the strawberry is shown in the display panel 16. Other buttons 20,22 are provided as user controls to manipulate and organise photographs stored in the memory of the camera.

The camera 10 is also provided with a detector 24 for capturing the scent 26 of the subject 18. The detector 24 is operated by a second button 28, the pressing of which causes the detector 24 to analyse the scent 26. If a specific object is held close to the detector 24 then the scent of the object will be captured, whilst if the detector is simply exposed to the ambient environment then the smell of the surroundings will be analysed instead. The detector 24 comprises a device operable to perform a chemical analysis of local gas (i.e. air). Such scent analysis devices (often known as 'electronic noses') are already known in the art, although to date they have not been incorporated in portable cameras.

Scent capture devices such as these typically comprise either a gas chromatography device, a mass spectrometry device or a chemical sensor, the construction of which are known to those skilled in the art. Once captured, the chemical compositional analysis (or 'fingerprint') of the scent 26 is stored in the camera along with the image it accompanies.

As well as still cameras, video cameras may also be provided with a scent analyser as described above. Such a camera would be configured to capture the ambient scent every minute or so during recording, or altematively at specific times as judged by the director (e.g. at the beginning of each new scene). Such a recording may then be played back to viewers in a suitably equipped cinema (as suggested in US 6,152,829).

As Figure 1 also illustrates, an image and its accompanying scent may be downloaded 29 from the camera 10 to a PC 30. The image may then be displayed on the display 32 of the computer. In the example shown in Figure 1 the PC is also provided with a scent generation device 34 such as the iSmell Personal Scent Synthesizer as produced by DigiScents Inc., which may be used to produce a scent 36 corresponding to the scent 26 that was previously captured. The scent 36 may then be perceived by a user whilst viewing the image on the computer display 32. The scent 36 provides the user with an evocative reminder of the subject of the photograph, thereby enhancing his recollection of the moment when the photograph was taken.

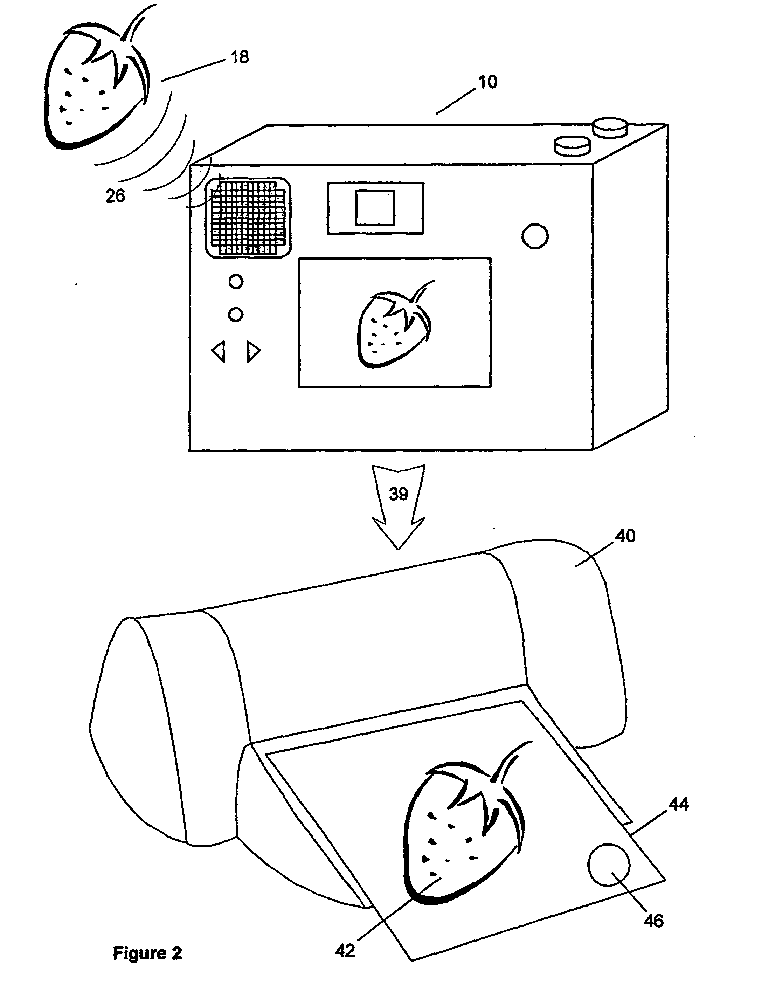

Figure 2 illustrates the same camera as in Figure 1, but arranged to download 39 the image and scent of the object to an ink-jet printer 40. As well as printing the image 42, the printer 40 also transfers a synthesised scent of the object 18 onto the paper 44. This may be achieved by providing the printer 40 with a cartridge containing a number of basic (or 'prime') scents, which are then combined in appropriate proportions to generate the required scent on delivery onto the paper. These prime scents are analogous to prime colours, in that a few prime scents may be combined to enable a range of scents to be produced. Accordingly, the scent 26 captured by the camera 10 may be synthesised by the printer and deposited onto the paper 44. Techniques for mixing prime scents to achieve a synthesised scent are known to those skilled in the art, as exemplified by the DigiScents devices (although these do not extend to depositing scents onto paper). Mixing of the prime scents may occur 'in-flight' - i.e. between leaving the scent cartridge and reaching the paper - or alternatively may be pre-mixed prior to delivery to the paper. The entire of the page may be coated with the synthesised scent, or alternatively the scent may just be sprayed onto a specific region 46 of the paper. Delivery of the scent may occur prior to deposition of the ink (i.e. to impregnate the paper in advance), or following the ink. The region 46 may be outside of the area of the page that is occupied by the image, in order to prevent the scented chemicals from merging with the ink. After deposition of the scent to the region 46, this region may subsequently be covered with a thin scratchable coating to give a 'scratch and sniff' panel, such that the scent may be released when the panel is scratched (e.g. by a coin). The use of a 'scratch and sniff panel provides a means by which the scent may be released a plurality of times from the picture, potentially over an extended period of time.

Other methods for printing scented images are disclosed in US 5,975,675 (Kim), WO 00/79346 A1 (Levy) and EP 1,002,840 A1 (NCR).

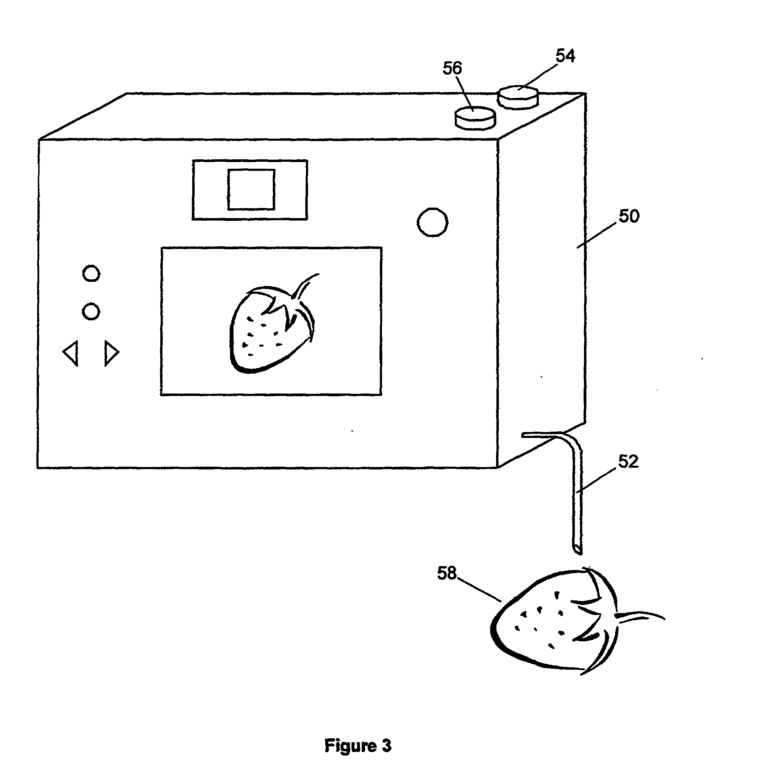

The present invention is intended to cover the sense of taste as well as smell. Figure 3 shows a digital camera 50 similar to the previous camera 10, but with a probe 52 instead of the scent detector 24. Alongside the image capture button 54 is a taste capture button 56 which, when pressed, causes the probe 52 to perform its analysis.

In use, the probe 52 is inserted into an object, typically a foodstuff such as a strawberry 58 as shown in the Figure. The object 58 will usually be the subject of the photograph, but need not necessarily be so. On inserting the probe 52 and pressing the button 56 the camera performs an analysis of the taste of the object 58.

The concept of taste is fundamentally scent coupled with one or more of the taste sensations of 'sweet', 'sour', 'bitter' and 'salt'. In practice, each of these is detected by the human tongue, whilst the scent is smelled essentially by the nose. The analysis performed by the camera is therefore predominantly one of the scent of the object 58, employing gas chromatography, mass spectrometry or chemical sensing techniques as described above. Since the object 58 is most likely to be solid or liquid, a small sample is taken up the probe 52 under vacuum and is passed into a heating chamber in which it is then vapourised. The gaseous form of the sample is then analysed by gas chromatography, mass spectrometry or a solid state chemical sensor. The associated concepts of sweet, sour, bitter and salt are also analysed by chemical analysis. The relative proportions of the chemicals responsible for these four sensations are recorded in the camera, together with the analysis of the chemical fingerprint of the scent.

Instead of using a probe for taste analysis, the camera may be provided with an analysis chamber into which a sample of the object 58 is manually placed. The sample is then vapourised and analysed as described above.

When the taste analysis has been completed, any solid, liquid or gaseous matter from the sample remaining in the camera is expelled.

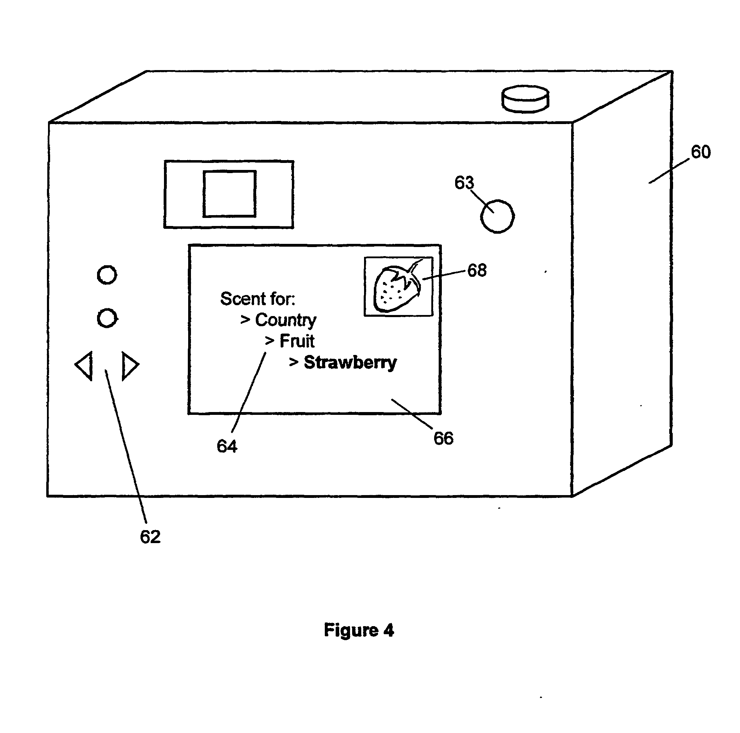

It will be appreciated that the apparatus required to analyse scents and tastes is highly complex and may therefore result in a relatively expensive product. Furthermore, scent synthesis technology may only allow a certain number of scents and aromas to be generated. In light of these shortcomings, a less expensive camera is shown in Figure 4. Here, the camera 60 is based around a conventional digital camera, and does not incorporate any chemical analysis equipment. However, the user controls 62,63 are configured and operable such that a scent (or taste) may be selected from a hierarchical menu 64 displayed on the display panel 66 on the rear of the camera. The options given in this menu correspond to, and are limited by, the scents that may subsequently be reproduced by the scent synthesis equipment.

The scent or taste is selected to accompany the image 68 that has been taken using the camera. The scent may well be chosen to be that of the subject of the photograph (as is the case with the strawberry in the Figure), although a scent may be chosen that is radically different from the subject. This gives rise to the possibility of devising amusing combinations of images and scents. For example, the scent of a farmyard may be selected to accompany a photograph of a beloved family member. The scent may be specified, potentially at any time, after the photograph has been taken. The scent specified by the user is then stored along with the photograph, and is transferred to the PC or printer for subsequent synthesis using the techniques described previously.

As has been mentioned above, scents and aromas experienced by a person at an event can serve as powerful reminders of the event. However, at the time of taking a photograph, the ambient scent may not correspond to any of the scents available for selection by the user of the camera of Figure 4. Accurate scent analysis equipment may not be available (e.g. because it is unaffordable). In such instances it is possible to utilise scent synthesis at the point of capturing the image, in order to provide stimulus for subsequent triggering of the user's memory. The scents that are able to be synthesised at the time of image capture correspond to those that are available for subsequent post-event reproduction. The user's memory relates post-event synthesis of the scent with the production of the same scent at the time of the event, and this thereby enhances the user's recollection of the event itself. Accordingly the user's memory is triggered by stimulus association.

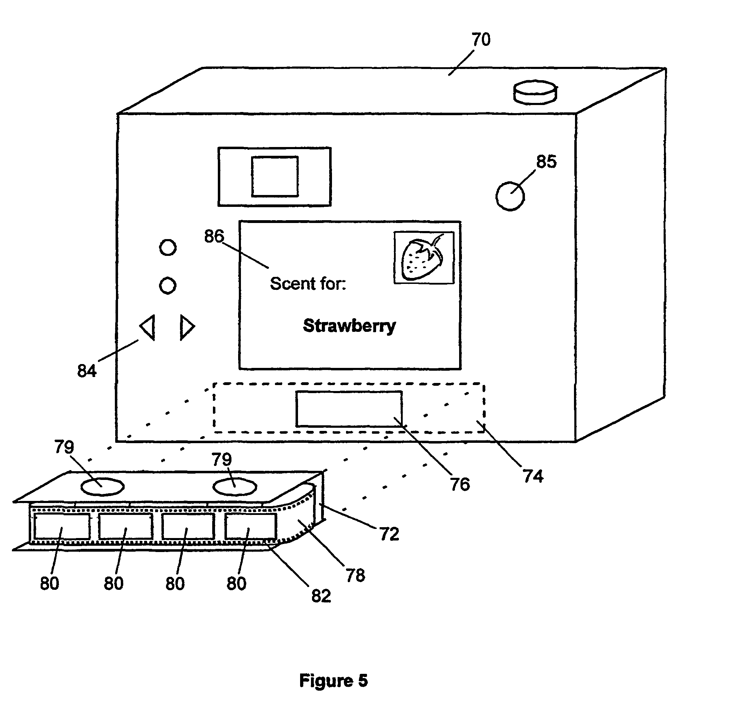

A camera equipped to release a scent at the point of image capture is illustrated in Figure 5. The camera 70 is provided with a cassette drive mechanism adapted to receive a cassette 72. The cassette 72 is loaded into the rear of the camera through a door 74 provided with an aperture 76. The door 74 is dosed after the cassette has been loaded. The cassette 72 contains a tape 78 wound between two spools 79, and along the tape 78 are a plurality of differently scented regions or segments 80. These regions 80 may also take the form of self-adhesive scented stickers, thereby enabling transfer of the scent from the tape onto a printed image or elsewhere. The aperture 76 is so shaped and sized to allow a scented region 80 to be open to a user. The tape also has strips of encoded digital information 82 (e.g. magnetic data) readable by a detector within the camera, which enable the tape to be controllably spooled by the camera.

In use, the user identifies his desired scent on the display screen 86 by operating the user controls 84 and then specifies his choice by pressing the 'select' button 85. The scent options available through the display 86 correspond to each of the different scented regions 80. The tape is then spooled by the camera 70, using the digital encoding 82, so as to position the desired scented panel by the aperture 76. (Alternatively the camera may spool the tape in accordance with each and every press of the user controls 86.)

Having selected a scented region 80 using the user controls 86, the scent is then released into the environment to provide the user with olfactory stimulation at the point of image capture. The scent may be emitted by the camera gently heating the chosen scented panel on the tape (e.g. by passing an electric current through a local heating element positioned level with the aperture 76). Alternatively the scented regions on the tape may be immediately smellable, or may be of a 'scratch and sniff' variety such that the user is required to scratch the chosen one through the aperture 76 for its scent to be released. (Alternatively it may be possible to configure the camera to scratch the panel using mechanical means in order to cause the scent to be released.)

Different cassettes 72 may be produced specific to certain locations or activities. For example, a cassette may be made that predominantly contains scents of the Orient (e.g. spices and Oriental cookery), whilst another might contain scents found on the seashore (e.g. tar, seafood, and smoke from a ferry).

The scent selected for emission at the point of image capture using the camera is intended to be re-emitted when subsequently viewing the image, in order to enhance recollection of the event. The cassette 72 that was used at the point of image capture may therefore be transferred from the camera 70 to a compatible device such as a computer peripheral or stand-alone printer, equipped with similar means for operating the cassette and for enabling the scent to be released.

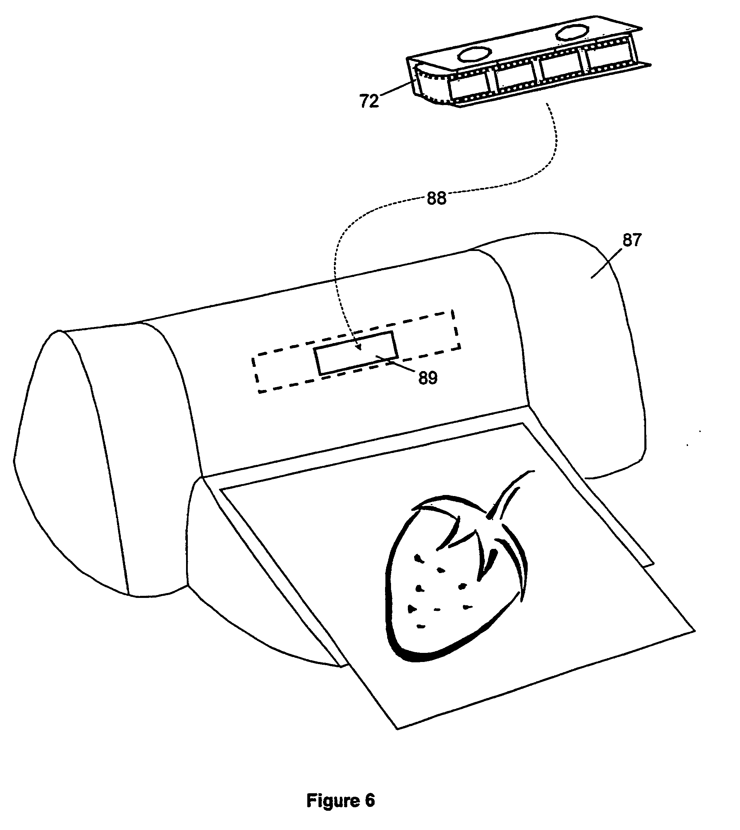

An example of such a printer is shown in Figure 6. The printer 87 comprises a cassette drive mechanism adapted to receive the cassette 72 from the camera of Figure 5. The cassette 72 is transferred 88 to the printer 87 and inserted such that the scented regions 80 can pass in front of the aperture 89. In use, typically under the control of a PC or a digital camera, the printer 87 will print the image and also spool the tape 78 in order to position the previously-specified scented region before the aperture 89. Internally the printer 87 may comprise means as described previously herein for locally heating the desired scented panel to such that the scent is emitted. If the scented regions are of the form of self-adhesive stickers then the printer may be configured to transfer the selected scented sticker onto the printed image during the printing process.

It will be appreciated that a device for operating such a scent generation cassette may be made independent from a printer. For example, a computer peripheral device may be constructed that interfaces with a PC and is operable to generate scents from a cassette on instruction from the PC.

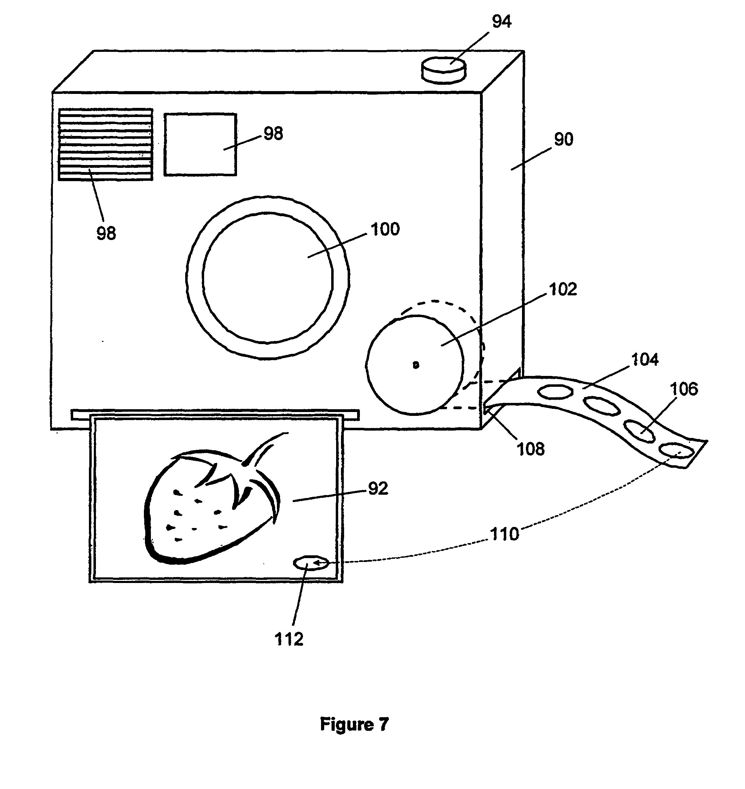

Figure 7 illustrates a simpler camera that allows a scent to be experienced at the point of image capture. In this embodiment the camera 90 is equipped with printing means so as to substantially instantly produce a printed photograph 92. The camera 90 is equipped with standard features of an image capture button 94, a flash 96, a viewfinder 98 and a lens 100. However, as Figure 7 shows, the camera 90 is also provided with a spool 102 of tape 104 on which self-adhesive scented stickers 106 are stuck. The spool 102 is contained within the camera 90 and emerges through an aperture 108 in a face of the camera. A range of pre-prepared scented stickers 106 are provided on the spool 102, and the user is required to transfer 110 a sticker of their choice from the tape 104 to the printed photograph 92. The transfer 110 of the selected scented sticker to the photograph would primarily be a manual operation, although it is envisaged that a camera could also be constructed in which the chosen sticker is automatically peeled from the tape 104 and transferred to the photograph 92 on printing. The scented stickers may be of a 'scratch and sniff' kind, to facilitate controlled release of the scent as and when desired, over an extended period of time.

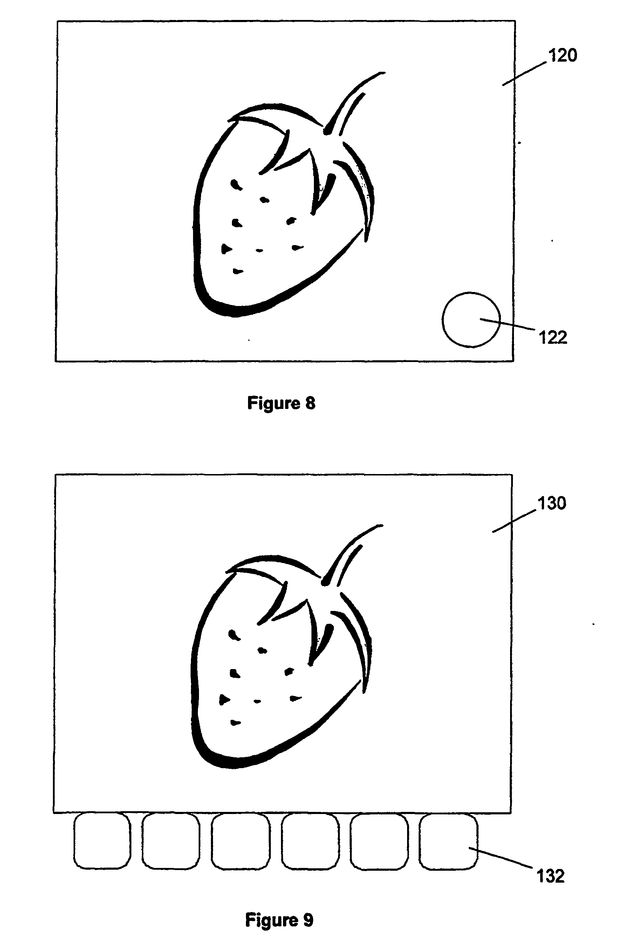

An example of a scent-enabled photograph is given in Figure 8. The photograph 120 has a scented region 122 which may be a scented sticker transferred from a camera as in Figure 7, or a scented region deposited from a suitably equipped printer as described previously.

The issue of conveying taste to a user will now be considered in more detail. Since taste is detected by the tongue, e.g. through licking or eating, it will be appreciated that the substances involved must be safe and non-toxic. With that proviso, a printer configured to deposit a scent on a page may readily be adapted to use lickable scented chemicals, together with non-toxic ink. The photograph as a whole may thereby be made lickable, or alternatively just a specific lickable region (e.g. 122 of Figure 8). Similarly, stickers (e.g. 80 from Figure 5, or 106 from Figure 7) may be made lickable.

If edible paper (e.g. rice paper) is used in the printer, together with non-toxic ink and scent, then the whole photograph may be made edible. However, it will of course be appreciated that an edible photograph can only be consumed once. To overcome this shortfall, a photograph as shown in Figure 9 may be produced which comprises a number of edible tabs 132 (e.g. made of rice paper) attached to the photograph 130 itself. The required scent or taste is applied to these tabs 132, which can then be detached and eaten (or licked) as required. Rice paper is ideal for making edible photographs or tabs, since it has essentially no flavour itself and therefore adopts the flavour of whatever flavouring is deposited onto it.

A third party service (e.g. operating via the internet) may be used to manufacture edible photographs using image and scent data supplied by a user, and then send the finished article to the user via conventional postal delivery means.

## Claims

1. A camera comprising image capture means (14) operable to capture an image, integral scent association means (24, 28) operable by a user to associate a scent with the captured image, **characterised by** integral means for causing the said scent to be perceived by a user.

2. A camera as claimed in Claim 1 wherein the scent association means comprise a detector or probe (52) connected to the camera (50), the detector being operable to analyse the scent of its surrounding medium (58).

3. A camera as daimed in Claim 2 wherein the detector or probe (52) comprises a gas chromatography device, a mass spectrometry device or a chemical sensor.

4. A camera as claimed in Claim 1 wherein the scent association means comprise a user interface (62) electrically coupled to processing means and a display (66), configured and operable to allow a user to select a scent from a menu (64).

5. A camera as claimed in any preceding Claim being a digital camera and comprising transfer means (29) operable to transfer an image and data representing the scent to processing means (30) remote from the camera.

6. A camera as claimed in Claim 5 wherein the transfer means are selected from a group comprising: a port configured to receive and electrically couple with a cable, wireless digital communication means, infra-red digital communication means, radio frequency communication means.

7. A camera as claimed in Claim 1 or Claim 4 further comprising a plurality of specimens of scents (80).

8. A camera as daimed in Claim 7 when dependent on Claim 4, wherein the specimens (80) correspond to the contents of the said menu (64) and the camera is configured to present a specimen of scent to the user in accordance with a selection made using the said menu (64).

9. A camera as claimed in Claim 8, or in Claim 7 when dependent on Claim 1, wherein the specimens (80, 106) are provided on a spool of tape (78, 102) operable by a user.

10. A camera as daimed in Claim 9 wherein each of the said specimens (80, 106) comprises a scented region covered by a scratchable coating, whereby scratching of the coating releases the scent from the underlying region.

11. A camera as claimed in Claim 10 wherein each of the said specimens is edible.

12. A camera as claimed in any of Claims 7 to 11 wherein the specimens are configured to be affixed (110) to a printed photograph (92).

13. A camera as claimed in Claim 12 wherein the specimens (106) have a self-adhesive surface.

14. A camera as claimed in Claim 13 having an integral printer.

15. A camera as claimed in Claim 14 configured to affix (110) a specimen onto a photograph (92) on printing.

16. A method for delivering a scent with an image, comprising using a camera to capture an image and the further steps of:
using the camera to associate a scent with the image;
**characterised by** the step of using means integral with the camera to cause the said scent to be perceived by a user.

17. A method as claimed in Claim 16 wherein step (a) comprises using a detector or probe (52) connected to the camera to analyse the scent of the surrounding medium (58).

18. A method as claimed in Claim 16 wherein step (a) comprises operating a user interface (62) to select a scent from a menu (64) provided on a display (66) on the camera.

19. A method as claimed in any of Claims 16 to 18 using a digital camera and further comprising transferring the image, and data representing the said scent, to processing means (30) remote from the camera.

20. A method as claimed in Claim 19 comprising electronically transferring the image, and the data representing the said scent, to a device selected from a group comprising: a printer (40) equipped with scent generation means, a personal computer (30) equipped with scent generation means; and then using the said scent generation means to generate the said scent for perception by a user.

21. A method as claimed in Claim 16 or Claim 18 further comprising providing a plurality of specimens of scents (80) accessible from the camera from which the user can specify a choice.

22. A method as daimed in Claim 21 when dependent on Claim 18, wherein the specimens (80) correspond to the contents of the menu (64) and step (b) comprises the camera presenting a specimen (80) to the user in accordance with the selection made by the user using the menu (64) in step (a).

23. A method as claimed in Claim 22, or in Claim 21 when dependent on Claim 16, wherein step (b) comprises providing the specimens (80, 106) on a spool of tape (78, 102) accessible by the user, from which specimens the user may specify a choice.

24. A method as claimed in Claim 23 further comprising transferring (88) the spool of tape to a separate device (87) from which the scent of the specified specimen may be perceived by a user.

25. A method as claimed in Claim 23 further comprising warming the specified specimen to cause the emission of its scent.

26. A method as claimed in Claim 23 further comprising applying (110) the selected specimen to the printed image (92).

## Patentansprüche

1. Eine Kamera, die eine Bildaufnahmeeinrichtung (14), die wirksam ist, um ein Bild aufzunehmen, und eine integrierte Geruchszuordnungseinrichtung (24, 28) umfasst, die durch einen Benutzer betreibbar ist, um dem aufgenommenen Bild einen Geruch zuzuordnen, **gekennzeichnet durch** eine integrierte Einrichtung zum Bewirken, dass der Geruch **durch** einen Benutzer wahrgenommen wird.

2. Eine Kamera gemäß Anspruch 1, bei der die Geruchszuordnungseinrichtung einen Detektor oder eine Sonde (52) umfasst, die mit der Kamera (50) verbunden ist, wobei der Detektor wirksam ist, um den Geruch des umgebenden Mediums (58) zu analysieren.

3. Eine Kamera gemäß Anspruch 2, bei der der Detektor oder die Sonde (52) eine Gaschromatographievorrichtung, eine Massenspektrometrievorrichtung oder einen chemischen Sensor umfasst.

4. Eine Kamera gemäß Anspruch 1, bei der die Geruchszuordnungseinrichtung eine Benutzerschnittstelle (62), die elektrisch mit der Verarbeitungseinrichtung gekoppelt ist, und eine Anzeige (66) umfasst, die konfiguriert und wirksam ist, um es einem Benutzer zu ermöglichen, einen Geruch von einem Menü (64) auszuwählen.

5. Eine Kamera gemäß einem der vorhergehenden Ansprüche, die eine Digitalkamera ist und eine Übertragungseinrichtung (29) umfasst, die wirksam ist, um ein Bild und Daten, die den Geruch darstellen, zu der Verarbeitungseinrichtung (30) entfernt von der Kamera zu übertragen.

6. Eine Kamera gemäß Anspruch 5, bei der die Übertragungseinrichtung von einer Gruppe ausgewählt ist, die Folgendes umfasst: ein Tor, das konfiguriert ist, um ein Kabel aufzunehmen und elektrisch mit demselben zu koppeln, eine drahtlose digitale Kommunikationseinrichtung, eine Infrarotdigitalkommunikationseinrichtung, eine Hochfrequenzkommunikationseinrichtung.

7. Eine Kamera gemäß Anspruch 1 oder Anspruch 4, die ferner eine Mehrzahl von Proben von Gerüchen (80) umfasst.

8. Eine Kamera gemäß Anspruch 7 in Rückbezug auf Anspruch 4, wobei die Proben den Inhalten des Menüs (64) entsprechen und die Kamera konfiguriert ist, um dem Benutzer eine Probe des Geruchs gemäß einer Auswahl zu präsentieren, die unter Verwendung des Menüs (64) getroffen wurde.

9. Eine Kamera gemäß Anspruch 8 oder Anspruch 7 in Rückbezug auf Anspruch 1, wobei die Proben (80, 106) auf einer Bandspule (78, 102) geliefert werden, die durch einen Benutzer betrieben werden kann.

10. Eine Kamera gemäß Anspruch 9, bei der jede der Proben (80, 106) eine duftende Region umfasst, die durch eine kratzbare Beschichtung bedeckt ist, wobei das Kratzen der Beschichtung den Duft von der darunter liegenden Region freigibt.

11. Eine Kamera gemäß Anspruch 10, bei der jede der Proben essbar ist.

12. Eine Kamera gemäß einem der Ansprüche 7 bis 11, bei der die Proben konfiguriert sind, um an eine gedruckte Photographie (92) angebracht (110) zu werden.

13. Eine Kamera gemäß Anspruch 12, bei der die Proben (106) eine selbsthaftende Oberfläche haben.

14. Eine Kamera gemäß Anspruch 13, die einen integrierten Drucker aufweist.

15. Eine Kamera gemäß Anspruch 14, die konfiguriert ist, um beim Drucken eine Probe auf eine Photographie (92) anzubringen (110).

16. Ein Verfahren zum Liefern eines Geruchs mit einem Bild, das das Verwenden einer Kamera zum Aufnehmen eines Bildes umfasst und den weiteren Schritt des
Verwendens der Kamera zum Zuordnen eines Geruchs zu dem Bild;
**gekennzeichnet durch** den Schritt des Verwendens einer Einrichtung integriert mit der Kamera, um zu bewirken, dass der Geruch **durch** einen Benutzer wahrgenommen wird.

17. Ein Verfahren gemäß Anspruch 16, bei dem Schritt (a) das Verwenden eines Detektors oder einer Sonde (52) umfasst, die mit der Kamera verbunden ist, um den Geruch des umgebenden Mediums (58) zu analysieren.

18. Ein Verfahren gemäß Anspruch 16, bei dem Schritt (a) das Betreiben einer Benutzerschnittstelle (60) umfasst, um einen Geruch von einem Menü (64) auszuwählen, das auf einer Anzeige (66) auf der Kamera bereitgestellt ist.

19. Ein Verfahren gemäß einem der Ansprüche 16 bis 18, das eine Digitalkamera verwendet und ferner das Übertragen des Bildes und der Daten, die den Geruch darstellen, an die Verarbeitungseinrichtung (30) entfernt von der Kamera umfasst.

20. Ein Verfahren gemäß Anspruch 19, das das elektronische Übertragen des Bildes und der Daten, die den Geruch darstellen, an eine Vorrichtung umfasst, die aus einer Gruppe ausgewählt ist, die aus Folgendem besteht: einem Drucker (40), der mit einer Geruchserzeugungseinrichtung ausgestattet ist, einem Personalcomputer (30), der mit einer Geruchserzeugungseinrichtung ausgestattet ist;
und dann Verwenden der Geruchserzeugungseinrichtung zum Erzeugen des Geruchs für die Wahrnehmung durch einen Benutzer.

21. Ein Verfahren gemäß Anspruch 16 oder 18, das ferner das Bereitstellen einer Mehrzahl von Proben von Gerüchen (80) umfasst, die von der Kamera aus zugreifbar sind, von der der Benutzer eine Auswahl spezifizieren kann.

22. Ein Verfahren gemäß Anspruch 21 in Rückbezug auf Anspruch 18, bei dem die Proben (80) den Inhalten des Menüs (64) entsprechen und Schritt (b) umfasst, dass die Kamera dem Benutzer eine Probe (80) präsentiert, gemäß der Auswahl, die durch den Benutzer unter Verwendung des Menüs (64) in Schritt (a) getroffen wurde.

23. Ein Verfahren gemäß Anspruch 22 oder 21 in Rückbezug auf Anspruch 16, wobei Schritt (b) das Bereitstellen der Proben (80, 106) auf einer Bandspule (78, 102) umfasst, auf die der Benutzer zugreifen kann, wobei der Benutzer von diesen Proben eine Auswahl spezifizieren kann.

24. Ein Verfahren gemäß Anspruch 23, das ferner das Übertragen (88) der Bandspule zu einer getrennten Vorrichtung (87) umfasst, von der der Geruch der spezifizierten Probe durch einen Benutzer wahrgenommen werden kann.

25. Ein Verfahren gemäß Anspruch 23, das ferner das Erwärmen der spezifizierten Probe umfasst, um das Verströmen des Geruchs derselben zu bewirken.

26. Ein Verfahren gemäß Anspruch 23, das ferner das Aufbringen (110) der ausgewählten Probe auf das gedruckte Bild (92) umfasst.

## Revendications

1. Appareil photographique comprenant des moyens de capture d'image (14) pouvant être mis en oeuvre pour capturer une image, des moyens d'association de parfum intégrés (24, 28) pouvant être mis en oeuvre par un utilisateur pour associer un parfum à l'image capturée, **caractérisé par** des moyens intégrés pour faire en sorte que ledit parfum soit perçu par un utilisateur.

2. Appareil photographique selon la revendication 1, dans lequel les moyens d'association de parfum comprennent un détecteur ou une sonde (52) connecté à l'appareil photographique (50), le détecteur pouvant être mis en oeuvre pour analyser le parfum de son milieu ambiant (58).

3. Appareil photographique selon la revendication 2, dans lequel le détecteur ou la sonde (52) comprend un dispositif de chromatographie en phase gazeuse, un dispositif de spectrométrie de masse ou un capteur chimique.

4. Appareil photographique selon la revendication 1, dans lequel les moyens d'association de parfum comprennent une interface utilisateur (62) couplée électriquement à des moyens de traitement et un afficheur (66), configurée et pouvant être mise en oeuvre pour permettre à un utilisateur de sélectionner un parfum dans un menu (64).

5. Appareil photographique selon l'une quelconque des revendications précédentes consistant en un appareil photographique numérique et comprenant des moyens de transfert (29) pouvant être mis en oeuvre pour transférer une image et des données représentant le parfum à des moyens de traitement (30) à distance de l'appareil photographique.

6. Appareil photographique selon la revendication 5, dans lequel les moyens de transfert sont sélectionnés dans un groupe comprenant : un port configuré pour recevoir et être couplé électriquement à un câble, des moyens de communication numérique sans fil, des moyens de communication numérique infrarouge, des moyens de communication radiofréquence.

7. Appareil photographique selon la revendication 1 ou la revendication 4, comprenant en outre une pluralité d'échantillons de parfums (80).

8. Appareil photographique selon la revendication 7 lorsqu'elle dépend de la revendication 4, dans lequel les échantillons (80) correspondent aux contenus dudit menu (64) et l'appareil photographique est configuré pour présenter un échantillon de parfum à l'utilisateur conformément à une sélection effectuée en utilisant ledit menu (64).

9. Appareil photographique selon la revendication 8, ou la revendication 7 lorsqu'elle dépend de la revendication 1, dans lequel les échantillons (80, 106) sont fournis sur une bobine de bande (78, 102) pouvant être mise en oeuvre par un utilisateur.

10. Appareil photographique selon la revendication 9, dans lequel chacun desdits échantillons (80, 106) comprend une région parfumée revêtue d'un revêtement pouvant être gratté, moyennant quoi le fait de gratter le revêtement libère le parfum de la région sous-jacente.

11. Appareil photographique selon la revendication 10, dans lequel chacun desdits échantillons est comestible.

12. Appareil photographique selon l'une quelconque des revendications 7 à 11, dans lequel les échantillons sont configurés pour être apposés (110) à une photographie (92) imprimée.

13. Appareil photographique selon la revendication 12, dans lequel les échantillons (106) comportent une surface autoadhésive.

14. Appareil photographique selon la revendication 13 comportant une imprimante intégrée.

15. Appareil photographique selon la revendication 14 configuré pour apposer (110) un échantillon sur une photographie (92) lors d'une impression.

16. Procédé pour délivrer un parfum avec une image, comprenant l'utilisation d'un appareil photographique pour capturer une image et l'étape supplémentaire consistant à :
utiliser l'appareil photographique pour associer un parfum à l'image ;
**caractérisé par** l'étape consistant à utiliser des moyens intégrés à l'appareil photographique pour faire en sorte que ledit parfum soit perçu par un utilisateur.

17. Procédé selon la revendication 16, dans lequel l'étape (a) comprend l'utilisation d'un détecteur ou d'une sonde (52) connecté à l'appareil photographique pour analyser le parfum du milieu ambiant (58).

18. Procédé selon la revendication 16, dans lequel l'étape (a) comprend la mise en oeuvre d'une interface utilisateur (62) pour sélectionner un parfum dans un menu (64) fourni sur un écran (66) sur l'appareil photographique.

19. Procédé selon l'une quelconque des revendications 16 à 18 utilisant un appareil photographique numérique et comprenant, en outre, le transfert de l'image, et de données représentant ledit parfum, à des moyens de traitement (30) à distance de l'appareil photographique.

20. Procédé selon la revendication 19 comprenant le transfert électroniquement de l'image, et des données représentant ledit parfum, à un dispositif sélectionné dans un groupe comprenant : une imprimante (40) équipée de moyens de génération de parfum, un ordinateur personnel (30) équipé de moyens de génération de parfum ;
et l'utilisation ensuite desdits moyens de génération de parfum pour générer ledit parfum pour sa perception par un utilisateur.

21. Procédé selon la revendication 16 ou la revendication 18 comprenant, en outre, la fourniture d'une pluralité d'échantillons de parfums (80) accessibles à partir de l'appareil photographique à partir duquel l'utilisateur peut spécifier un choix.

22. Procédé selon la revendication 21 lorsqu'elle dépend de la revendication 18, dans lequel les échantillons (80) correspondent aux contenus du menu (64) et l'étape (b) comprend la présentation à l'utilisateur, par l'appareil photographique, d'un échantillon (80) conformément à la sélection effectuée par l'utilisateur en utilisant le menu (64) à l'étape (a).

23. Procédé selon la revendication 22, ou la revendication 21 lorsqu'elle dépend de la revendication 16, dans lequel l'étape (b) comprend la fourniture des échantillons (80, 106) sur une bobine de bande (78, 108) accessible par l'utilisateur, parmi lesquels échantillons l'utilisateur peut spécifier un choix.

24. Procédé selon la revendication 23 comprenant, en outre, le transfert (88) de la bobine de bande à un dispositif séparé (87) à partir duquel le parfum de l'échantillon spécifié peut être perçu par un utilisateur.

25. Procédé selon la revendication 23 comprenant, en outre, le chauffage de l'échantillon spécifié afin de provoquer l'émission de son parfum.

26. Procédé selon la revendication 23 comprenant, en outre, l'application (110) de l'échantillon sélectionné à l'image (92) imprimée.
